Europäisches Patentamt
European Patent Office
Office européen des brevets

(19)

(11) Publication number: **0 398 665 A1**

(12) **EUROPEAN PATENT APPLICATION**

(21) Application number: **90305247.0**

(22) Date of filing: **15.05.90**

(51) Int. Cl.5: **C07D 311/58, C07D 491/052, A61K 31/44, //(C07D491/052, 311:00,221:00)**

The title of the invention has been amended (Guidelines for Examination in the EPO, A-III, 7.3).

(30) Priority: **17.05.89 GB 8911280**

(43) Date of publication of application: **22.11.90 Bulletin 90/47**

(84) Designated Contracting States: **ES GR**

(71) Applicant: **Beecham Group p.l.c.**
**SB House Great West Road**
**Brentford Middlesex TW8 9BD(GB)**

(72) Inventor: **Pinto, Ivan Leo, Beecham Pharmaceuticals**
**Great Burgh, Yew Tree Bottom Road**
**Epsom, Surrey KT18 5XQ(GB)**
Inventor: **Stemp, Geoffrey, Beecham Pharmaceuticals**
**Coldharbour Road, The Pinnacles, Fourth Avenue**
**Harlow, Essex CM19 5AD(GB)**

(74) Representative: **Rutter, Keith et al**
**Smith Kline Beecham, Corporate Patents,**
**Great Burgh, Yew Tree Bottom Road**
**Epsom Surrey KT18 5XQ(GB)**

(54) **Benzopyran derivatives.**

(57) A compound of formula (I):

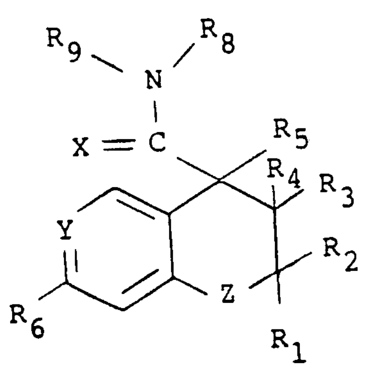

(I)

or, where appropriate, a pharmaceutically acceptable salt, ester or amide thereof, or a pharmaceutically acceptable solvate thereof
wherein:
X represents O or S;
Y represents N or $N^{+}$-$O^{-}$ or a moiety $CR_7$ wherein
$R_7$ is as defined below;
Z represents O, $CH_2$, NR, $S(O)_n$ or Z represents a bond;
R represents hydrogen, alkyl or alkylcarbonyl
$R_1$ and $R_2$ independently represent hydrogen or alkyl; or

EP 0 398 665 A1

$R_1$ and $R_2$ together represent a $C_{2-7}$ polymethylene chain;
$R_3$ represents hydrogen, hydroxy, alkoxy or acyloxy;
$R_4$ is hydrogen;
$R_5$ is hydrogen or $R_4$ and $R_5$ together represent a bond;
$R_6$ represents hydrogen, substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)$-CH-, $R^dC(=N.OH)$-, $R^dC(=N.NH_2)$- or alkenyl optionally substituted by alkylcarbonyl, nitro or cyano;
when Y represents $CR_7$, $R_7$ is independently selected from the variables mentioned above in relation to $R_6$ providing that at least one of $R_6$ or $R_7$ is other than hydrogen;
$R^a$ represents $R^d$ or $R^dO$- and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, hydroxy, halo, haloalkyl, nitro and cyano;
$R^b$ and $R^c$ each independently represent hydrogen, alkyl or alkylcarbonyl;
T represents a bond or $T^1$, $T^1$ represents -CS- or $T^2$ and
$T^2$ represents -CO-, -SO- or $-SO_2-$ and
$R_8$ represents hydrogen, alkyl or substituted or unsubstituted aryl;
$R_9$ represents hydrogen or alkyl, substituted or unsubstituted aryl or $R_8$ and $R_9$ together represent a $C_{3-7}$ polymethylene group; and
n represents 0, 1 or 2; a process for preparing such a compound, a pharmaceutical composition containing such a compound and the use of such a compound or composition in medicine.

## NOVEL COMPOUNDS

This invention relates to certain novel compounds, in particular to 4-(aminothiocarbonyl and aminocarbonyl)benzopyran derivatives having smooth muscle relaxant activity, to processes for their preparation, to compositions containing such compounds and to the use of such compounds and compositions in medicine.

EP-A-76075, 93535, 95316, 107423, 120426, 126311, 126350, 126367 and 138134 describe certain benzopyran derivatives having inter alia antihypertensive activity. EP-A-176689 also discloses that certain benzopyran derivatives are useful for the treatment of inter alia disorders of the respiratory system.

A group of 4-(aminothiocarbonyl and aminocarbonyl)benzopyran derivatives has now been discovered which surprisingly has smooth muscle relaxant activity, and such compounds are therefore potentially useful as bronchodilators in the treatment of disorders of the respiratory tract, such as reversible airways obstruction and asthma, and also in the treatment of hypertension. Such compounds are also indicated to be of potential use in the treatment of disorders associated with smooth muscle contraction of the gastro-intestinal tract, uterus or the urinary tract including the ureter. Such disorders respectively include irritable bowel syndrome and diverticular disease; premature labour; incontinence; renal cholic and disorders associated with the passage of kidney stones. They are also indicated as of potential use in the treatment of cardiovascular disorders other than hypertension, such as congestive heart failure, angina, peripheral vascular disease and cerebral vascular disease; and also in the treatment and/or prophylaxis of disorders associated with pulmonary hypertension and of disorders associated with right heart failure.

These compounds are also indicated to have potential use as anti-convulsants in the treatment of epilepsy.

Accordingly, the present invention provides a compound of formula (I):

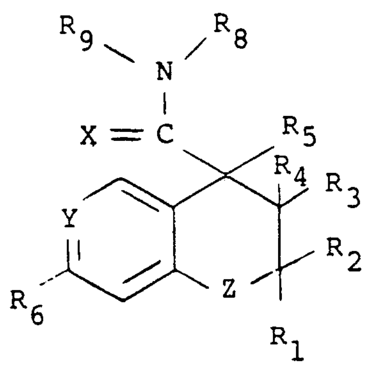

(I)

or, where appropriate, a pharmaceutically acceptable salt, ester or amide thereof, or a pharmaceutically acceptable solvate thereof

wherein:

X represents O or S;

Y represents N or $N^{+}$-$O^{-}$ or a moiety $CR_7$ wherein

$R_7$ is as defined below;

Z represents O, $CH_2$, NR, $S(O)_n$ or Z represents a bond;

R represents hydrogen, alkyl or alkylcarbonyl

$R_1$ and $R_2$ independently represent hydrogen or alkyl; or

$R_1$ and $R_2$ together represent a $C_{2-7}$ polymethylene chain;

$R_3$ represents hydrogen, hydroxy, alkoxy or acyloxy;

$R_4$ is hydrogen;

$R_5$ is hydrogen or $R_4$ and $R_5$ together represent a bond;

$R_6$ represents hydrogen, substituted or unsubstituted

alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(=N.NH_2)$- or alkenyl optionally substituted by alkylcarbonyl, nitro or cyano;

when Y represents $CR_7$, $R_7$ is independently selected from the variables mentioned above in relation to $R_6$ providing that at least one of $R_6$ or $R_7$ is other than hydrogen;

$R^a$ represents $R^d$ or $R^dO$- and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, hydroxy, halo, haloalkyl, nitro and cyano;
$R^b$ and $R^c$ each independently represent hydrogen, alkyl or alkylcarbonyl;
T represents a bond or $T^1$, $T^1$ represents -CS- or $T^2$ and
$T^2$ represents -CO-, -SO- or $-SO_2$- and
$R_8$ represents hydrogen, alkyl or substituted or unsubstituted aryl;
$R_9$ represents hydrogen or alkyl, substituted or unsubstituted aryl or $R_8$ and $R_9$ together represent a $C_{3-7}$ polymethylene group; and
n represents 0, 1 or 2.

Suitably, X represents O.

Preferably, S represents S.

Suitably, when Y is N or $N^+-O^-$, $R_6$ is hydrogen.

Suitably, Y is N or $N^+-O^-$.

Suitably, Y is $CR_7$.

Suitably, when Y is $CR_7$, one of $R_6$ and $R_7$ is hydrogen and the other is selected from substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$, $R^dCS.O$-, $R^d(OH)CH$-, $R^d(SH)CH$-, $R^dC(=N.OH)$, $R^dC(=N.NH_2)$- or alkenyl optionally substituted by alkylcarbonyl, nitro or cyano, wherein $R^a$, $R^b$, $R^c$, $R^d$, T, $T^1$ and $T^2$ are as defined in relation to formula (I).

Favourably, one of $R_6$ and $R_7$ is hydrogen and the other is selected from alkyl, substituted alkyl, nitro or cyano.

Preferably, $R_6$ represents hydrogen, Preferably, $R_7$ represents alkyl, for example ethyl, fluoroalkyl, for example pentafluoroethyl, or cyano.

In particular $R_7$ represents cyano.

When $R_6$ or $R_7$ represents a substituted alkyl group, suitable optional substituents include one or more of halogen, hydroxy, alkoxy, thiol, nitro, cyano and alkylcarbonyl, especially halogen, in particular fluorine.

Preferably, $R_1$ and $R_2$ are both $C_{1-6}$ alkyl, and in particular $R_1$ and $R_2$ are both methyl.

Preferably, $R_3$ represents hydroxy.

Suitably, $R_4$ and $R_5$ each represent hydrogen.

Suitably, $R_4$ and $R_5$ together represent a bond.

Suitably, $R_8$ or $R_9$ represents hydrogen, $C_{1-6}$ alkyl, phenyl or substituted phenyl,

Favoured values for $R_8$ or $R_9$ include hydrogen, $C_{1-6}$ alkyl or phenyl and especially $C_{1-6}$ alkyl or phenyl.

Preferably, $R_8$ represents hydrogen.

Preferred values for $R_9$ include $C_{1-6}$ alkyl or phenyl.

Suitable $C_{1-6}$ alkyl values of $R_9$ include methyl, ethyl, propyl and butyl, examples being methyl and butyl.

When $R_8$ or $R_9$ represents substituted aryl, it is preferably substituted phenyl, especially monosubstituted phenyl for example 4-substituted phenyl. Examples of substituents are halogen and alkoxy, particular examples of $R_8$ or $R_9$ are 4-fluorophenyl and 4-methoxyphenyl.

Preferably, Z represents O.

Suitable heteroaryl groups include 5- or 6-membered monocyclic or 9- or 10-membered bicyclic heteroaryl groups, preferably 5- or 6-membered monocyclic heteroaryl groups.

Preferred 5- or 6-membered monocyclic or 9- or 10-membered bicyclic heteroaryl groups are those containing one, two or three heteroatoms selected from the class of oxygen, nitrogen and sulphur and which, in the case of there being more than one heteroatom, are the same or different.

Suitable 5- or 6-membered monocyclic heteroaryl moieties include furyl, thienyl, pyrrolyl, oxazolyl, thiazolyl, imidazolyl and thiadiazolyl, and pyridyl, pyridazyl, pyrimidyl, pyrazyl and triazyl. Preferred 5- or 6-membered heteroaryl groups include furyl, thienyl, pyrrolyl and pyridyl, in particular 2- and 3-furyl, 2- and 3-pyrrolyl 2- and 3-thienyl, and 2-, 3-and 4-pyridyl.

Suitable 9- or 10-membered bicyclic heteroaryl moieties include benzofuryl, benzothienyl, indolyl and indazolyl, quinolinyl and isoquinolinyl, and quinazolinyl. Preferred 9- or 10- membered bicyclic heteroaryl groups include 2- and 3-benzofuryl, 2- and 3-benzothienyl, and 2- and 3-indolyl, and 2- and 3-quinolinyl.

Suitable aryl groups are phenyl or naphthyl groups.

Suitable substituents for any aryl group include one or more groups or atoms selected from alkyl, alkoxy, hydroxy, halogen, fluoroalkyl, nitro, cyano, carboxy or an ester thereof, alkylcarbonyloxy, amino, monoalkylamino, dialkylamino, aminocarbonyl, monoalkylaminocarbonyl or dialkylaminocarbonyl.

In any optionally substituted aryl, or optionally substituted heteroaryl group, the preferred number of substituents is 1, 2, 3 or 4.

Preferred substituents for any substituted aryl or heteroaryl group include methyl, methoxy, hydroxy, chloro, fluoro, nitro or cyano.

When used herein the term "halogen" refers to fluorine, chlorine, bromine and iodine; preferably fluorine.

Suitably alkyl groups, or alkyl groups forming part of other groups such as in the alkoxy group, are $C_{1-12}$ alkyl groups especially $C_{1-6}$ alkyl groups e.g. methyl, ethyl, n-propyl, iso-propyl, n-butyl, isobutyl or tert-butyl groups.

Unless mentioned to the contrary, suitable optional substituents for any alkyl group includes those substituents indicated above for the aryl group.

Suitably alkenyl groups are $C_{2-12}$ groups especially $C_{2-6}$ alkenyl groups.

Suitable alkynyl groups are $C_{2-12}$ alkynyl groups especially $C_{2-6}$ alkynyl groups.

Suitable polymethylene groups include $C_3$, $C_4$, $C_5$, $C_6$ and $C_7$ polymethylene groups.

Suitable acyloxy groups include alkylcarbonyloxy groups wherein the alkyl group is as defined above.

Suitable pharmaceutically acceptable salts of the compounds of formula (I) include acid addition salts and salts of carboxy groups.

Examples of pharmaceutically acceptable acid addition salts of the compounds of formula (I) includes acid addition salts of optionally substituted amino groups, such as the hydrochloride and hydrobromide salts. It will also be appreciated that when Y in the compound of formula (I) represents N, then the resulting pyridine moiety may yield acid addition salts, such as the hydrochloride or hydrobromide salts.

Examples of pharmaceutically acceptable salts of carboxy groups include metal salts, such as alkali metal salts, or optionally substituted ammonium salts.

Suitable pharmaceutically acceptable esters include esters of carboxy groups and imino esters of the moiety $-C(=X)NR_8R_9$.

Examples of pharmaceutically acceptable esters of carboxy groups are esters such as $C_{1-6}$ alkyl esters.

Examples of pharmaceutically acceptable imino esters of the moiety $-C(=X)NR_8R_9$ are imino esters of formula $-C(=NR^p)OR^q$ wherein $R^p$ is $C_{1-6}$ alkyl or phenyl and $R^q$ is $C_{1-6}$ alkyl.

Examples of pharmaceutically acceptable amides of carboxyl groups include amides such as amides of formula $-CO.NR^sR^t$ wherein $R^s$ and $R^t$ each independently represent hydrogen or $C_{1-6}$ alkyl.

The compounds of formula (I) may also exist in the form of solvates, preferably hydrates, and the invention extends to such solvates.

The compounds of formula (I), may exist in the form of optical isomers. For example chirality is present in those compounds of formula (I) wherein $R_3$ is hydrogen, hydroxy, alkoxy or acyloxy and $R_4$ and/or $R_5$ is hydrogen and those wherein $R_1$ and $R_2$ are different. The present invention extends to all optical isomers of the compounds of formula (I) whether in the form of single isomers or of mixtures thereof, such as racemates.

The compounds of formula (I) may also exist in geometrical isomeric forms all of which are encompassed by the present invention, including those wherein the moiety $X=\underset{|}{C}-NR_8R_9$ and $R_3$ are disposed either mutually cis with respect to one another or, preferably, mutually trans with respect to one another.

Particular examples of compounds of formula (I) include the compounds prepared in the Examples hereinafter.

The present invention also provides a process for the preparation of a compound of formula (I) or, where appropriate a pharmaceutically acceptable salt, ester or amide thereof, or a pharmaceutically acceptable solvate thereof, which comprises reacting a compound of formula (II):

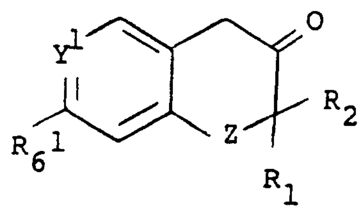

(II)

wherein $R_1$, $R_2$, and Z are as defined in relation to formula (I), $Y^1$ represents Y or a moiety convertible thereto and $R_6{}^1$ represents $R_6$ or a moiety convertible thereto, with a compound of formula (III):

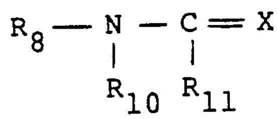

(III)

wherein $R_8$ and X are as defined in relation to formula (I),$R_{10}$ represents $R_9$ as defined above and $R_{11}$ represents a leaving group, or $R_{11}$ together with $R_{10}$ represents a bond; to provide a compound of formula (I) wherein $R_3$ represents hydroxy and $R_4$ together with $R_5$ represents a bond, and optionally thereafter converting such a compound into a compound of formula (I) wherein $R_3$ represents hydroxy and $R_4$ and $R_5$ both represent hydrogen and thereafter, if required, converting such a compound into a compound of formula (I) wherein $R_3$ represents hydrogen and $R_4$ and $R_5$ together represent a bond; and thereafter, if required, carrying out one or more of the following optional steps:

(i) converting one compound of formula (I) into another compound of formula (I);

(ii) preparing a pharmaceutically acceptable salt, ester or amide of a compound of formula (I); or

(iii) preparing a pharmaceutically acceptable solvate of a compound of formula (I) or a solvate thereof.

When $R_{11}$ represents a leaving group, a suitable leaving group is a halogen atom such as a chlorine atom.

The reaction between a compound of formula (II) and a compound of formula (III) may be carried out in any suitable aprotic solvent, for example tetrahydrofuran, at any convenient temperature providing a suitable rate of formation of the required product, suitably ambient temperature, and preferably in the presence of a base.

Suitable bases are alkali metal $C_{1-4}$-alkoxides such as potassium t-butoxide.

A compound of formula (II) may be prepared by reacting a compound formula (IV):

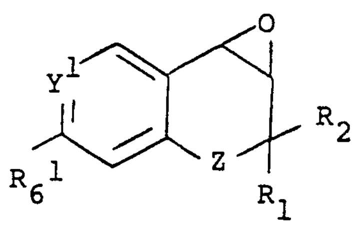

(IV)

wherein $R_1$, $R_2$, $R_6{}^1$, $Y^1$ and Z are as defined in relation to formula (II), with an appropriate Lewis acid.

An appropriate Lewis acid is boron trifluoride or a mild Lewis acid, such as magnesium bromide.

The reaction between the compound of formula (IV) and the Lewis acid may be carried out in any suitable inert solvent, such as toluene, at any convenient temperature providing a suitable rate of formulation of the required product, generally an elevated temperature such as in the range of from 40° to 110°C, for example at 80°C.

A compound of formula (I) wherein $R_3$ represents hydroxy and $R_4$ together with $R_5$ represent a bond may be converted into a compound of formula (I) wherein $R_3$ represents hydroxy and $R_4$ and $R_5$ both represent hydrogen by reduction, using any suitable reduction method, such as a complex metal hydride reduction using, for example, potassium borohydride in methanol.

Suitably, a compound of formula (I) wherein $R_3$ represents hydroxy and $R_4$ and $R_5$ both represent hydrogen (A) may be converted into a compound of formula (I) wherein $R_3$ is hydrogen and $R_4$ together with $R_5$ represent a bond (B) by activating the $C_3$- hydroxy group of the trans-isomer of (A) - for example by conversion to a mesylate by treatment with mesyl chloride in the presence of triethylamine - and forming the appropriate compound (B) by trans -elimination -for example by treatment of the said mesylate with a base such as potassium t-butoxide in an aprotic solvent such as tetrahydrofuran.

In one particular aspect, the present invention provides a process for preparing a compound of formula (I) wherein $R_3$, $R_4$ and $R_5$ each represent hydrogen, or where appropriate a pharmaceutically acceptable

salt, ester or amide thereof, or a pharmaceutically acceptable solvate thereof, which process comprises reacting a compound of formula (V):

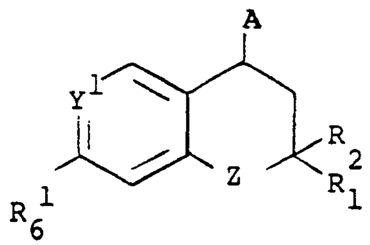

(V)

wherein $R_1$, $R_2$, $R_6{}^1$, $Y^1$ and Z are as defined in relation to formula (II) and A represents a group or atom convertible to $-CX.NR_8R_9$,
with an appropriate reagent capable of converting A into $-CX.NR_8R_9$, and thereafter if required carrying out one or more of the following optional steps:

(i) converting one compound of formula (I) into another compound of formula (I);

(ii) preparing a pharmaceutically acceptable salt, ester or amide of a compound of formula (I); or

(iii) preparing a pharmaceutically acceptable solvate of a compound of formula (I) or a solvate thereof.

Conveniently, A represents $-CO.A_1$ wherein $A_1$ is OH or a halogen atom. Favourably A is a halogen atom, for example a chlorine atom.

The specific nature of the appropriate reagent capable of converting A into $-CX.NR_8R_9$, depends upon the particular nature of A and in general the appropriate conventional reagent is employed, for example when A represents $-CO.A_1$, especially when $A_1$ is halogen, the appropriate reagent is an amine of formula (VI):

$HNR_8R_9$ (VI)

wherein $R_8$ and $R_9$ are as defined in relation to formula (I).

The reaction between a compound of formula (V) and the appropriate reagent may be carried out under the appropriate conventional conditions, for example the reaction between the compounds of formulae (V) and (VI) may be carried out in any suitable solvent, such as dichloromethane, at a temperature which provides a suitable rate of formation of the required product, conveniently at ambient temperature.

Where $A_1$ in reagent (VI) represents OH, the OH group may be converted into a halogen atom by reaction with a suitable halogenating agent, such as an oxalyl halide, for example oxalyl chloride, and the resulting product reacted in-situ with the compound of formula (V), to provide a compound of formula (I).

The halogenation of the compound of formula (VI) may be carried out using conventional halogenation conditions, for example in an inert solvent, such as dichloromethane, at an elevated temperature, conveniently the reflux temperature of the solvent.

Compounds of formula (V) are either known compounds or they may be prepared by conventional methods from known compounds, for example a compound of formula (V) wherein A represents -COOH may be prepared by hydrolysing a compound of formula (VII):

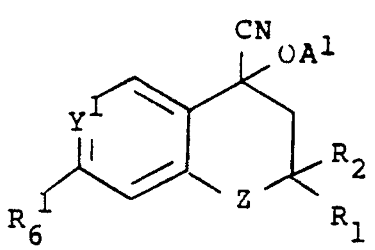

(VII)

wherein $R_1$, $R_2$, $R_6{}^1$, $Y^1$ and Z are as defined in relation to a compound of formula (V) and $A^1$ represents OH, $O^-M^+$ wherein $M^+$ is an alkali metal counter ion, or preferably, $OSiMe_3$.

Suitable methods of hydrolysing a compound of formula (VII) include conventional methods such as treatment with stannous chloride/hydrochloric acid at an elevated temperature, such as in the range of from 100 to 150°C, in a solvent such as acetic acid.

A compound of formula (VII) may be prepared by reacting a compound of formula (VIII):

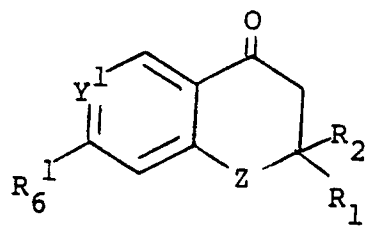

(VIII)

wherein $R_1$, $R_2$, $R_6'$, $Y'$, and Z are as defined in relation to formula (V), with a source of cyanide ions, such as an alkali metal cyanide or, preferably, trimethylsilyl cyanide.

The reaction between the compound of formula (VIII) and the source of cyanide ions may be carried out in any suitable solvent at any temperature providing a suitable rate of formation of the required product. Suitably, when trimethylsilyl cyanide is the source of cyanide ions it may also be used as the reaction solvent, the reaction then generally proceeds at ambient temperature. Preferably, the reaction between compound (VIII) and the source of cyanide ions, such as trimethylsilyl cyanide, is carried out in the presence of a Lewis acid such as zinc iodide.

Suitably $R_6'$ represents $R_6$. Suitably $Y^1$ represents Y.

Suitable conversions of a compound of formula (I) into another compound of formula (I) include:

(i) converting $R_3$ in the compound of formula (I) into another $R_3$;

(ii) thiating a compound of formula (I) wherein >C=X represents a >C=O group to provide a compound of formula (I) wherein >C=X represents a >C=S group; (iii) oxidising a compound of formula (I) wherein >C=X represents a >C=S group to provide a compound of formula (I) wherein >C=X represents a >C=O group.

Examples of an optional conversion of $R_3$ in a compound of formula (I) into another $R_3$ are generally known in the art. For example, when $R_3$ is hydroxy, it may be alkylated using any conventional alkylation method such as alkyl iodide in an inert solvent, such as toluene, in the presence of a base, such as sodium hydride, or, for methylation, trimethyloxonium tetrafluoroborate in an inert solvent such as dichloromethane, or the hydroxy groups may be acylated using a carboxylic acid chloride or the appropriate anhydride in a non-hydroxylic solvent, such as toluene or dichloromethane, in the presence of an acid acceptor such as triethylamine . Alternatively, when $R_3$ is $C_{1-6}$ alkoxy, it may be converted into hydroxy by any suitable dealkylation technique, for example by treatment with trimethylsilyl iodide in an aprotic solvent. Also when $R_3$ is $C_{1-7}$ acyloxy it may be converted into hydroxy by conventional hydrolysis using, for example a dilute mineral acid.

The thiation of >C=X group in a compound of formula (I) is suitably carried out with conventional thiation agents. such as hydrogen sulphide, phosphorus pentasulphide and Lawesson's reagent (p-methoxyphenylthiophosphine sulphide dimer).

The use of hydrogen sulphide and phosphorus pentasulphide may lead to side-reactions and, therefore, the use of Lawesson's reagent is preferred.

The thiation reaction conditions are conventional for the thiation agent employed. For example, the use of hydrogen sulphide is, preferably, acid catalysed by, for example, hydrogen chloride in a polar solvent, such as acetic acid or ethanol. The preferred use of

Lawesson's reagent is, preferably, carried out under reflux in a dry solvent, such as toluene or methylene chloride.

The oxidation of the >C=S group to a >C=O group may be carried out using any appropriate oxidation procedure, for example by using peroxide ion as oxidising agent, from any suitable source of peroxide ions. Suitable solvents are those used conventionally in the particular oxidation method chosen, for example an aprotic solvent such as tetrahydrofuran may be used with the source of peroxide ions.

In any of the abovementioned reactions suitable protecting groups may be used where required, such groups will be those conventional protecting groups that may be prepared and removed using known procedures and which protect the appropriate chemical group during the preparation of the required compounds.

Any conversion of $Y^1$ to Y or $R_6'$ to $R_6$ may be carried out using the appropriate conventional chemical procedure.

The optional formation of a pharmaceutically acceptable salt, when the resulting compound of formula (I) contains a salifiable group, may be carried out conventionally. Similarly, pharmaceutically acceptable esters, amides or solvates, for example hydrates, may be prepared using any convenient conventional

procedure.

Examples of methods for preparing an imino ester include treatment of the compound of formula (I) or a suitably protected form thereof, with a halogenating agent followed by an appropriate $C_{1-6}$ alkanol.

The compounds of formula (III) and (VI) are either known, commercially available compounds or they may be prepared according to methods used to prepare such compounds, for example those methods disclosed in Advanced Organic Chemistry, 3rd Edition, 1985, Wiley Interscience, March.

The compounds of formula (IV) and (VIII) are known compounds or they may be prepared according to analogous procedures used to prepare known compounds, for example those disclosed in the abovementioned European applications.

As mentioned previously, some of the compounds of formula (I) may exist in optically active forms, and the processes of the present invention produce mixtures of such forms. The individual enantiomers may be resolved by conventional methods.

It is preferred that the compounds of formula (I) are isolated in substantially pure form.

The compounds of formula (I), the pharmaceutically acceptable salts, ester or amide thereof or the pharmaceutically acceptable solvates thereof, have been found to have bronchodilator activity and/or blood-pressure lowering activity. They are therefore useful in the treatment of respiratory tract disorders, such as reversible airways obstruction, diverticular disease and asthma and also hypertension. They may also be of potential use in the treatment of other disorders hereinbefore described.

The present invention accordingly provides a pharmaceutical composition which comprises a compound of formula (I), or a pharmaceutically acceptable salt, ester or amide thereof or a pharmaceutically acceptable solvate thereof, and a pharmaceutically acceptable carrier.

The compositions are preferably adapted for oral administration. However, they may be adapted for other modes of administration, for example in the form of a spray, aerosol or other conventional method for inhalation, for treating respiratory tract disorders; or parenteral administration for patients suffering from heart failure. Other alternative modes of administration include sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns. Where appropriate, small amounts of other anti-asthmatics and bronchodilators, for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included.

The compositions may contain from 0.1% to 99% by weight, preferably from 10-60% by weight, of the active material, depending on the method of administration.

The present invention further provides a method of treatment of respiratory tract disorders or hypertension in mammals including man, which method comprises administering an effective, non-toxic amount of a compound of formula (I), or a pharmaceutically acceptable salt, ester or amide thereof or a pharmaceutically acceptable solvate thereof to the mammal in need thereof. The present invention also provides a method of treatment of disorders associated with smooth muscle contraction of the gastro-intestinal tract and the abovementioned cardiovascular disorders and epilepsy, in mammals including man, which method comprises administering an effective, non-toxic amount of a compound of formula (I), or a pharmaceutically acceptable salt, ester or amide thereof or a pharmaceutically acceptable solvate thereof to the mammal in need thereof.

An effective amount will depend on the relative efficacy of the compounds of the present invention, the severity of the respiratory tract disorder or hypertension being treated and the weight of the sufferer. However, a unit dose form of a composition of the invention may contain from 0.01 to 100mg of a compound of the invention (0.01 to 10mg via inhalation) and more usually from 0.1 to 50mg, for example 0.5 to 25mg such as 1, 2, 5, 10, 15 or 20mg. Such compositions may be administered from 1 to 6 times a day, more usually from 2 to 4 times a day, in a manner such that the daily dose is from 0.02 to 200mg for a 70 kg human adult and more particularly from 0.05to 100mg.

Suitable dosages for the treatment of disorders associated with smooth muscle contraction of the gastro intestinal tract and the abovementioned cardiovascular disorders and epilepsy are those described for the treatment of respiratory tract disorders.

In addition the present invention provides a compound of formula (I) or a pharmaceutically acceptable salt, ester or amide thereof, or a pharmaceutically acceptable solvate thereof, for use as an active therapeutic substance.

The present invention further provides a compound of formula (I) or a pharmaceutically acceptable salt, ester or amide thereof, or a pharmaceutically acceptable solvate thereof, for use in the treatment of respiratory tract disorders or hypertension or disorders associated with smooth muscle contraction of the gastro intestinal tract or the abovementioned cardiovascular disorders and epilepsy. The present invention further provides the use of a compound of formula (I), or a pharmaceutically acceptable salt, ester or amide thereof, or a pharmaceutically acceptable solvate thereof for the manufacture of a medicament for the treatment of respiratory tract disorders or hypertension or disorders associated with smooth muscle contraction of the gastro intestinal tract or the abovementioned cardiovascular disorders and epilepsy.

The following Procedures relate to the preparation of intermediates and the following Examples relate to the preparation of compounds of formula (I).

## Procedure 1

Trans-6-cyano-3,4-dihydro-2,2-dimethyl-4-methylaminothiocarbonyl-3-methylsulphonyloxy-2H-1-benzopyran

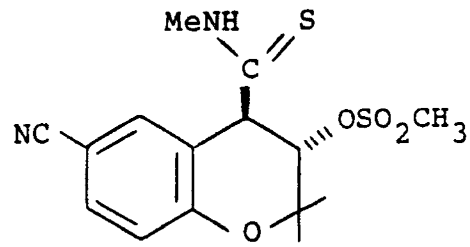

Methanesulphonylchloride (0.16ml, 2mmol) was added to a solution of trans-6-cyano-3,4-dihydro-2,2-dimethyl-4-methylaminothiocarbonyl-2H-1-benzopyran-3-ol (500mg, 1.81mmol) and triethylamine (0.33ml, 2.36mmol) in dichloromethane (20ml), and the solution stirred for 4.5 hours. The reaction was quenched with water, extracted with dichloromethane, the organics dried ($MgSO_4$) and concentrated to a solid (708mg). Chromatography on silica (chloroform) yielded trans-6-cyano-3,4- dihydro-2,2-dimethyl-4-methylaminothiocarbonyl-3-methylsulphonyloxy-2H-1-benzopyran (560mg, 88%), mp 160.5-161°C.

1H nmr ($CDCl_3$)

$\delta$ 1.33 (s,3H); 1.53 (s,3H); 3.13 (s,3H); 3.26 (d,3H, J=5Hz); 4.29 (d,1H, J=8Hz); 5.43 (d,1H, J=8Hz); 6.95 (d,1H, J=8.5Hz); 7.39 (d,1H, J=2Hz); 7.49 (dd,1H, J=2,8.5Hz); 7.70-7.72 (broad,1H).

Procedure 2

Trans-6-cyano-3,4-dihydro-2,2-dimethyl-4-t-butylaminothiocarbonyl-3-methylsulphonyloxy-2H-1-benzopyran

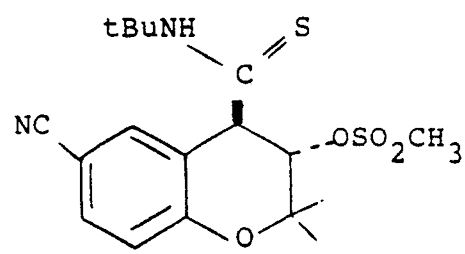

To a solution of trans-4-t-butylaminothiocarbonyl-6-cyano-3,4-dihydro-2,2-dimethyl-2H-1-benzopyran-3-ol Example 6 (0.23g, 0.72mmol) in methylene chloride (10ml) at 0°C was added triethylamine (0.13ml, 0.93mmol) followed by methane sulphonyl chloride (0.06ml, 0.78mmol) and the solution stirred for 16 hrs. The reaction mixture was poured into water, extracted with methylene chloride, dried and concentrated.

$^1$H nmr revealed only 50% reaction, therefore the mixture was redissolved in methylene chloride (10ml) and cooled to 0°C, whereupon triethylamine (0.15ml, 1.1mmol) and methanesulphonyl chloride (0.09ml, 1.2mmol) were added. The solution was stirred for 48 hrs then isolated as above to yield trans-4-t-butylaminothiocarbonyl-6-cyano-3,4-dihydro-2,2-dimethyl-3-methylsulphonyloxy-2H-1-benzopyran in quantitative yield.

Procedure 3

2,2-Dimethyl-3-oxo-2H-pyrano[3,2-c]pyridine

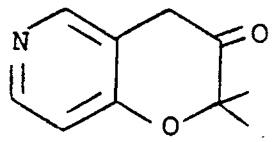

A mixture of 2,2-dimethyl-3,4-dihydro-3,4-epoxy-2H-pyrano[3,2-c]pyridine (1.2g, 7.3mmol) and borontrifluoride etherate (1.8ml, 14.6mmol) in toluene (70ml) was heated at 80°C for $\frac{1}{2}$ hr, allowed to cool, diluted with ethyl acetate and washed with saturated sodium bicarbonate solution. The organics were separated, dried and concentrated to yield 2,2-dimethyl-3-oxo-2H-pyrano[3,2-c]pyridine (650mg, 54%) which was not purified further.
$^1$H nmr ($CDCl_3$): δ 1.55 (s,6H); 3.85 (s,2H); 7.30 (m,2H); 8.50 (m, 1H).

Procedure 4

3,4-Dihydro-2,2-dimethyl-6-ethyl-2H-1-benzopyran-4-carboxylic acid

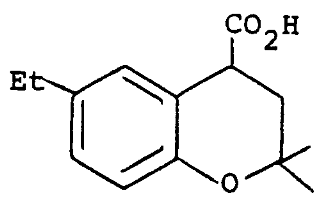

Tin (II) chloride dihydrate (4.5g, 20mmol), conc. hydrochloric acid (5ml) and 3,4-dihydro-2,2-dimethyl-6-ethyl-4-trimethylsilyloxy-2H-1-benzopyran-4-carbonitrile (1.5g, 5mmol) was dissolved in acetic acid (5ml) under $N_2$ and heated at 140° for 20 hr. The reaction mixture was cooled, diluted with dichloromethane, the organic phase washed with water, dried and concentrated to a brown oil which solidified on standing to give 3,4-dihydro-2,2-dimethyl-6-ethyl-2H-1-benzopyran-4-carboxylic acid (1.0g, 94%) as a solid, m.p. 109-110°C ($CHCl_3$/hexane).
$^1$H nmr ($CDCl_3$): δ 1.20 (t,J=7.5Hz,3H); 1.51 (s,3H); 1.57 (s,3H); 2.46 (dd, J=9.5, 2.5Hz,2H); 2.55 (q, J=7.5Hz,2H); 4.20 (t, J=9.5Hz,1H); 6.54 (brs,1H); 6.77 (d, J=8Hz,1H); 6.92 (d, J=2Hz,1H); 6.97 (dd, J=8,2Hz,1H); Found C, 71.61; H 7.83%. $C_{14}H_{18}O_3$ requires C, 71.8; H, 7.74%.

Procedure 5

3,4-Dihydro-2,2-dimethyl-6-ethyl-4-trimethylsilyloxy-2H-1-benzopyran-4-carbonitrile

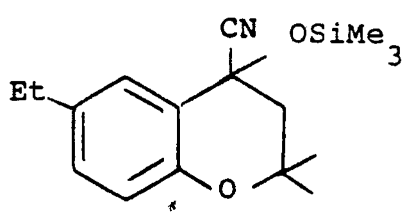

Trimethylsilyl cyanide (2.97ml, 30mmol) was added dropwise to a mixture of zinc iodide (64mg, 0.2mmol) and 3,4-dihydro-2,2-dimethyl-6-ethyl-2H-1-benzopyran-4-one (2.04g, 10mmol). The brown solution was stirred for 24 hrs, diluted with chloroform (100ml) and washed with potassium carbonate solution. The organic layer was dried and concentrated to give 3,4-dihydro-2,2-dimethyl-6-ethyl-4-trimethylsilyloxy-2H-1-benzopyran-4-carbonitrile (2.66g, 88%) as an oil.
$^1$H nmr (CDCl$_3$): δ 0.0 (s,9H); 0.98 (t, J=8Hz,3H); 1.16 (s,3H); 1.19 (s,3H); 2.12 (ABq, J=9.4Hz,2H); 2.34 (q, J=8Hz,2H); 6.50 (d, J=8.5Hz,1H); 6.85 (dd, J=8,2Hz,1H); 7.09 (d, J=2Hz,1H);
Accurate Mass Found 303.1649; $C_{17}H_{25}NO_2Si$ requires 303.1654.

Procedure 6

3,4-Dihydro-2,2-dimethyl-6-pentafluoroethyl-2H-1-benzopyran-3-one

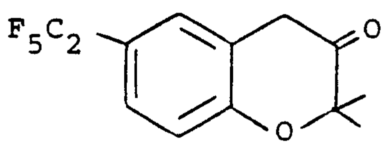

3,4-Dihydro-2,2-dimethyl-3,4-epoxy-6-pentafluoroethyl-2H-1-benzopyran (592mg, 2mmol) in toluene (6ml) was treated with dry powdered magnesium bromide etherate (580mg, 2.2mmol) under nitrogen and heated at 60°C for 16 hrs. The mixture was cooled, filtered and evaporated to yield 3,4-dihydro-2,2-dimethyl-6-pentafluoroethyl-2H-1-benzopyran-3-one (526mg, 89%).
$^1$H nmr (CDCl$_3$): δ 1.50 (s,6H); 3.70 (s,2H); 7.17 (d,1H, J=9Hz); 7.43 (bs,1H); 7.57 (bd,1H, J=9Hz).

Example 1

6-Cyano-2 ,2-dimethyl-4-methylaminothiocarbonyl-2H-1-benzopyran-3-ol

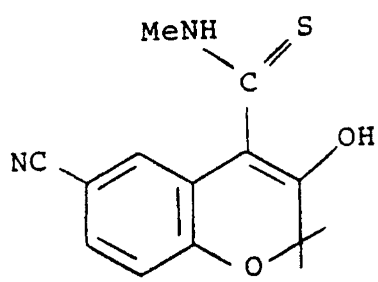

To potassium t-butoxide (323mg, 2.88mmol) in tetrahydrofuran (20ml) at 0°C under $N_2$ was added 6-cyano-3,4-dihydro-2,2-dimethyl-2H-1-benzopyran-3-one (580mg, 2.88mmol) in tetrahydrofuran (4ml). The solution was stirred at ambient temperature for 1 hour before addition of methylisothiocyanate (0.2ml, 2.9mmol) in tetrahydrofuran (1ml). After stirring for 4 hours, further methylisothiocyanate (0.05ml, 0.7mmol) was added and stirring continued for 18 hours. The reaction mixture was poured into 2N HCl (50ml), extracted with chloroform (3 x 50ml), the organics separated, dried ($MgSO_4$) and concentrated to a solid (758mg). Chromatography on silica (dichloromethane) gave 6-cyano-2,2-dimethyl-4-methylaminothiocarbonyl-2H-1-benzopyran-3-ol (425mg) m.p. 179.5-180.5°C (EtOAC).
$^1$H nmr (CDCl$_3$)
δ 1.47 (5, 6H); 3.26 (d, 3H, J=5Hz); 6.99 (d, 1H, J=8.5Hz); 7.35 (dd, 1H, J=8Hz, 2Hz); 7.47 (d, 1H, J=2Hz); 7.58 (8, 1H, broad); 13.46 (brs, 1H).

| | |
|---|---|
| Accurate Mass $C_{14}H_{14}N_2O_2S$ requires | 274.0776 |
| found | 274.0781 |

Examples 2 and 3

Trans-and Cis-6-cyano-3,4-dihydro-2,2-dimethyl-4-methylaminothiocarbonyl-2H-1-benzopyran-3-ol

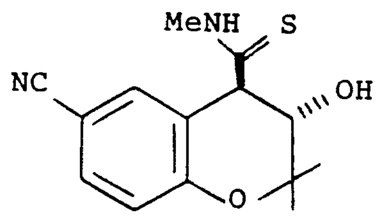

Example 2

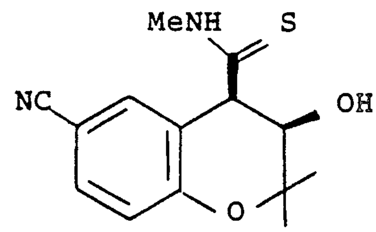

Example 3

Potassium borohydride (81mg, 1.50mmol) was added portionwise to a solution of 6-cyano-2,2-dimethyl-4-methylaminothiocarbonyl-2H-1-benzopyran-3-ol (375mg, 1.37mmol) in methanol (10ml), and the solution stirred for 1.5 hours. The reaction mixture was poured into water (100ml), extracted into chloroform (3 x 100ml), dried ($MgSO_4$), filtered and concentrated to an oil (365mg) which was chromatographed on silica. Elution with chloroform provided first cis-6-cyano-3,4-dihydro-2,2-dimethyl-4-methylaminothiocarbonyl-2H-1-benzopyran-3-ol (102mg, 27%), m.p. 170-171°C, followed by trans-6-cyano-3,4-dihydro-2,2-dimethyl-4-methylaminothiocarbonyl-2H-1-benzopyran-3-ol (140mg, 38%) m.p. 212-213°C.

Trans Isomer (Example 2)

$^1H$ nmr ($CDCl_3$):

$\delta$ 1.21 (s, 3H), 1.53 (s, 3H), 3.28 (d, 3H, J=5Hz collapses to singlet with $D_2O$), 3.31 (s, 1H disappears with $D_2O$); 4.02 (5, 2H changes to dd with $D_2O$), 6.92 (d, 1H, J=8.5Hz), 7.29 (d, 1H, J=2Hz), 7.45-7.53 (dd, 1H, J=8.5Hz, 2Hz + 1H, broad, disappears with $D_2O$).

| Anal: | | | | |
|---|---|---|---|---|
| Found, | C, 60.80; | H, 5.84; | N, 9.73% | |
| Requires, | C, 60.84; | H, 5.84; | N, 10.14% | for $C_{14}H_{16}N_2O_2S$ |

| | |
|---|---|
| Accurate mass $C_{14}H_{16}N_2O_2S$ requires | 276.0932 |
| found | 276.0928 |

Cis isomer (Example 3)

$^1H$ nmr ($CDCl_3$)

$\delta$ 1.29 (s,3H); 1.52 (s,3H); 3.25 (d, 3H, J=5Hz); 3.33 (br s, 1H disappears with $D_2O$); 4.07 (dd, 1H, J=3Hz, 5Hz collapses to d with $D_2O$); 4.51 (d, 1H, J=3Hz); 6.92 (d, 1H, J=8.5Hz), 7.43-7.47 (m, 2H); 8.23 (brs, 1H, disappears with $D_2O$).

| | |
|---|---|
| Accurate mass $C_{14}H_{16}N_2O_2S$ requires | 276.0932 |
| found | 276.0927. |

Example 4

6-Cyano-2,2-dimethyl-4-methylaminothiocarbonyl-2H-1-benzopyran

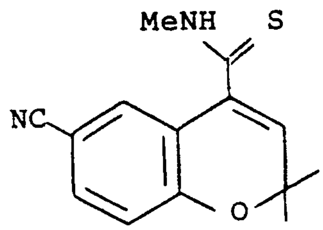

Potassium t-butoxide (99mg, 0.88mmol) was added portionwise to a solution of trans-6-cyano-3,4-dihydro-2,2-dimethyl-4-methylaminothiocarbonyl-3-methanesulphonyloxy-2H-1-benzopyran (280mg, 0.79mmol) in THF (20ml) at 0°C under $N_2$. The solution was stirred at 0°C for 3 hours, poured into water (100ml), extracted with diethyl ether (3x100ml), dried ($MgSO_4$) and concentrated to a gum (220mg). Chromatography on silica (chloroform) yielded 6-cyano-2,2-dimethyl-4-methylaminothiocarbonyl-2H-1-benzopyran (164mg, 80%), m.p. 174.5-175°C.

$^1H$ nmr ($CDCl_3$)

δ 1.49 (s, 6H); 3.28 (d, 3H, J = 5Hz); 5.86 (s, 1H) 6.85 (d, 1H, J = 8.5Hz); 7.41 (dd, 1H, J = 2, 8.5Hz); 7.71 (d, 1H, J = 2Hz + 1H broad).

| Anal: | | | | |
|---|---|---|---|---|
| $C_{14}H_{14}N_2O_2S$ requires | C, 65.09; | H, 5.46; | N, 10.85; | S = 12.41% |
| found | C, 64.88; | H, 5.42; | N, 10.67; | S, 12.19% |

Example 5

4-t-Butylaminothiocarbonyl-6-cyano-2,2-dimethyl-2H-1-benzopyran-3-ol

6-Cyano-3,4-dihydro-2,2-dimethyl-2H-1-benzopyran-3-one (1.0g, 4.97mmol) in THF (10ml) was added to a solution of potassium-t-butoxide (558mg, 4.97mmol) in THF (35ml) at 0°C under $N_2$. The solution was stirred for 2 hours at ambient temperature before addition of t-butylisothiocyanate (0.63ml, 4.97mmol) in THF (2ml). The reaction mixture was stirred at ambient temperature for 4 hours. Further potassium-t-butoxide (84mg, 0.75mmol) and t-butylisothiocyanate (0.1ml, 0.79mmol) were added and stirring continued for 18 hours. The reaction mixture was poured into 2N HCl (100ml), extracted with chloroform (3x100ml), dried ($MgSO_4$), filtered and concentrated in vacuo to a solid (1.43g) which was chromatographed on silica. Elution with choroform gave 4-t-butylaminothiocarbonyl-6-cyano-2,2-dimethyl-2H-1-benzopyran-3-ol (1.2g, 77%), m.p. 166-167°C.

$^1H$ nmr ($CDCl_3$)

δ 1.47 (s, 6H); 1.62 (s, 9H); 6.98 (d, 1H, J = 8Hz); 7.18 (br.s, 1H disappears with $D_2O$); 7.35 - 7.40 (m, 2H); 13.15 (s, iH dtsappears with $D_2O$).

| | |
|---|---|
| Accurate mass $C_{17}H_{20}N_2O_2S$ requires | 316.1245 |
| found | 316.1241 |

Examples 6 and 7

Trans- and Cis-4-t-butylaminothiocarbonyl-6-cyano-3,4-dihydro-2,2-dimethyl-2H-1-benzopyran-3-ol

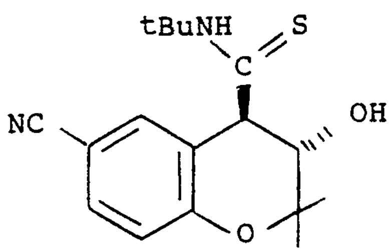

Example 6

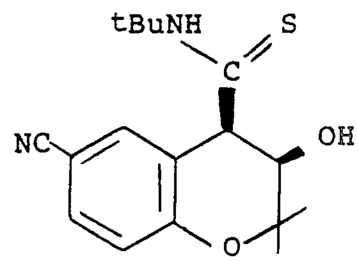

Example 7

Potassium borohydride (94mg, 1.74mmol) was added portionwise to a solution of 4-t-butylaminothiocarbonyl-6-cyano-2,2-dimethyl-2H-1-benzopyran-3-ol (500mg, 1.58mmol) in methanol (30ml) at 0°C, and the solution stirred for 3 hours at ambient temperature. Further potassium borohydride (10mg, 0.19mmol) was added and stirring continued for 1 hour. The reaction mixture was poured into water (100ml), extracted into chloroform (3 x 100ml), dried ($MgSO_4$) and concentrated to a solid (470mg). Chromatography on silica (dichloromethane) provided first cis-4-t-butylaminothiocarbonyl-6-cyano-3,4-dihydro-2,2-dimethyl-2H-1-benzopyran-3-ol (265mg, 53%) m.p. 167.5-168°C, followed by trans-4-t-butylaminothiocarbonyl-6-cyano-3,4- dihydro-2,2-dimethyl-2H-1-benzopyran-3-ol (95mg, 19%) m.p. 215-216°C.

Trans Isomer (Example 6)

$^1H$ nmr ($CDCl_3$) $\delta$ 1.20 (s, 3H); 1.50 (s, 3H); 1.64 (s, 9H); 3.88 (d, 1H, J=10Hz); 4.20 (br s, 1H, disappears with $D_2O$); 4.23 (d, 1H, J=10Hz); 6.82 (d, 1H, J=8.5Hz); 7.27 (d, 1H, J.2Hz); 7.38 (dd, 1H, J=2Hz, 8.5Hz); 8.67 (5, 1H).

| | |
|---|---|
| Accurate Mass $C_{17}H_{22}N_2O_2S$ requires | 318.1402 |
| found | 318.1398 |

Cis Isomer (Example 7)

$^1H$ nmr ($CDCl_3$)

$\delta$ 1.28 (s, 3H); 1.52 (s, 3H); 1.56 (s, 9H); 3.29 (brs, 1H, disappears with $D_2O$); 4.09 (d, 1H, J=3Hz); 4.32 (d, 1H, J=3Hz); 6.92 (d, 1H, J=8.5Hz); 7.45 (dd, 1H, J=8.5, 2Hz); 7.52 (d, 1H J=2Hz); 7.95 (brs, 1H).

| | |
|---|---|
| Accurate Mass $C_{17}H_{22}N_2O_2S$ requires | 318.1402 |
| found | 318.1401 |

Example 8

6-Cyano-2,2-dimethyl-4-phenylaminothiocarbonyl-2H-1-benzopyran-3-ol

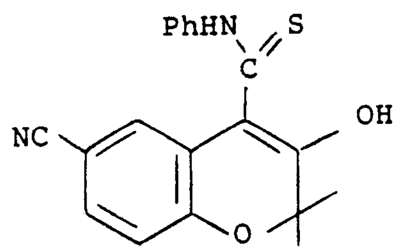

To potassium-t-butoxide (167mg, 1.5mmol) in THF (5ml) at 0°C, under $N_2$, was added 6-cyano-3,4-dihydro-2,2-dimethyl-2H-1-benzopyran-3-one (300mg, 1.49mmol) in THF (2ml). The solution was stirred at ambient temperature for 45 minutes whereupon phenylisothiocyanate (0.18ml, 1.49mmol) in THF (2ml) was added and the mixture stirred for a further 3 hours. The reaction was quenched with 2N HCl (50ml), extracted with chloroform (3x50ml) and the organics dried ($MgSO_4$) and concentrated to an orange solid (603mg). Chromatography on silica (dichloromethane) yielded 6-cyano-2,2-dimethyl-4-phenylaminothiocarbonyl-2H-1-benzopyran-3-ol (388mg, 78%).
1H nmr ($CDCl_3$)
$\delta$ 1.51 (s,6H); 7.01 (d,1H, J=8.5Hz); 7.35-7.69 (m,7H); 8.73 (s,1H, disappears with $D_2O$); 13.62 (s,1H, disappears with $D_2O$).

| Anal | | | |
|---|---|---|---|
| $C_{19}H_{16}N_2O_2S$ requires<br>found | C, 67.83;<br>C, 67.73; | H, 4.79;<br>H, 4.73; | N, 8.33%<br>N, 8.33% |

| Accurate Mass | |
|---|---|
| $C_{19}H_{16}N_2O_2S$ requires<br>found | 336.0932<br>336.0936 |

Example 9

6-Cyano-2 2-dimethyl-4-phenylaminocarbonyl-2H-1-benzopyran-3-ol

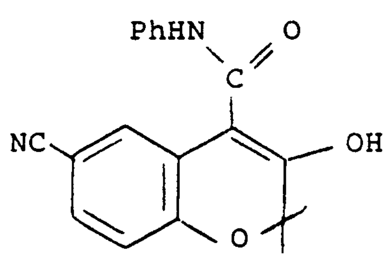

6-Cyano-3,4-dihydro-2,2-dimethyl-2H-1-benzopyran-3-one (2.5g, 12.4mmol) in THF (8ml) was added to a solution of potassium t-butoxide (1.53g, 13.63mmol) in THF (40ml) at 0°C under $N_2$.

The solution was stirred for 2 hours at ambient temperature before addition of phenylisocyanate (1.35ml, 12.4mmol) in THF (5ml). The reaction mixture was stirred for 3 hours at ambient temperature, poured into 2N HCl (100ml), extracted with ethyl acetate (3x100ml), dried ($MgSO_4$) and concentrated to a solid (4.4g) which was chromatographed on silica.

Elution with chloroform gave 6-cyano-2,2-dimethyl-4-phenylaminocarbonyl-2H-1-benzopyran-3-ol (2.2g, 55%) mp 171-172° (EtOAc).

$^1$H nmr [$CDCl_3$)

δ 1.50 (s,6H); 7.03 (d, 1H, J=8.5Hz); 7.18-7.26 (m, 1H); 7.25-7.6 (1H, broad, disappears with $D_2O$), 7.34-7.54 (m, 5H), 7.65 (d, 1H, J=2Hz); 14.05 (5, 1H disappears with $D_2O$).

| | | | |
|---|---|---|---|
| Anal $C_{19}H_{16}N_2O_2$ requires | C, 71.23; | H, 5.03; | N, 8.75%; |
| found | C, 71.23; | H, 5.00; | N, 8.75%; |

Examples 10 and 11

Cis and Trans-6-cyano-3,4-dihydro-2,2-dimethyl-4-phenylaminothiocarbonyl-2H-1-benzopyran-3-ol

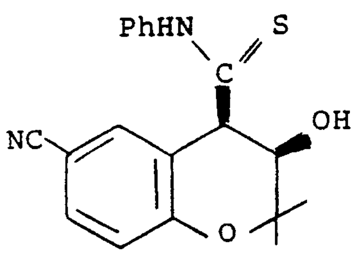

Example 10

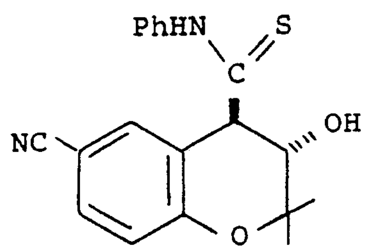

Example 11

Potassium borohydride (405mg, 7.5mmol) was added portionwise to a solution of 6-cyano-2,2-dimethyl-4-phenylaminothiocarbonyl-2H-1-benzopyran-3-ol (2.1g, 6.25mmol) in methanol (100ml) at 0°C. The solution was stirred for 3 hours whereupon further potassium borohydride (200mg, 3.75mmol) was added, and stirring continued for 18 hours at ambient temperature. The reaction mixture was poured into water (200ml), extracted with ethyl acetate (3x150ml), dried and concentrated to a solid. Chromatography on silica (dichloromethane/methanol, 99:1) provided cis-6-cyano-3,4-dihydro-2,2-dimethyl-4-phenylaminothiocarbonyl-2H-1-benzopyran-3-ol, (257mg), mp 201-2°C ($Et_2O$).

Further elution gave a mixture of cis and trans alcohols which was treated with 1,5-diazabicyclo [4.3.0] non-5-ene (6mg) in tetrahydrofuran under $N_2$ and the solution stirred for 3 hours.

The reaction was quenched with 2N HCl (50ml), extracted with diethyl ether (3x30ml), dried ($MgSO_4$) and concentrated to a gum (198mg). Purification by preparative HPLC (70% methanol, 30% water - reverse phase) gave trans-6-cyano-3,4-dihydro-2,2-dimethyl-4-phenylaminothiocarbonyl-2H-1-benzopyran-3-ol as a foam, mp = 90-93°C.

Cis isomer (Example 10)

$^1$H nmr ($CDCl_3$)

δ 1.33 (s,3H); 1.56 (s,3H); 2.90 (br,1H, disappears with $D_2O$); 4.21 (d,1H, J=3Hz); 4.66 (d,1H, J=3Hz); 6.97 (d,1H, J=8.5Hz); 7.25-7.31 (m,1H); 7.39-7.51 (m,3H); 7.68-7.71 (m,3H); 9.67 (br s,1H, disappears with $D_2O$).

| Accurate mass $C_{19}H_{18}N_2O_2S$ requires | 338.1089 |
|---|---|
| found | 338.1095 |

Trans isomer (Example 11)
$^1H$ nmr ($CDCl_3$)
$\delta$ 1.26 (s,3H); 1.56 (s,3H); 3.22 (s,1H, disappears with $D_2O$); 4.13 (d,1H, J=9.5Hz); 4.20 (d,1H, J=9.5Hz); 6.93 (d,1H, J=8Hz); 7.32-7.48 (m,5H); 7.69 (d,2H, J=8Hz); 8.96 (1H, broad, disappears with $D_2O$).

| Accurate mass $C_{19}H_{18}N_2O_2S$ requires | 338.1089 |
|---|---|
| found | 338.1096 |

Example 12

6-Cyano-2,2-dimethyl-4-(4-Fluorophenylaminothiocarbonyl)-2H-1-benzopyran-3-ol

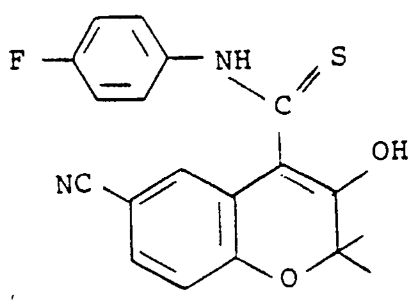

(E12)

6-Cyano-3,4-dihydro-2,2-dimethyl-2H-1-benzopyran-3-one (1g, 4.97mmol) in tetrahydrofuran (10ml) was added to a solution of potassium-t-butoxide (586mg, 5.22mmol) in tetrahydrofuran (30ml) at 0°C under $N_2$. The solution was stirred for 1 hour at ambient temperature before addition of 4-fluoro- phenylIsothiocyanate (760mg, 4.97mmol) in tetrahydrofuran (5ml). The reaction mixture was stirred at ambient temperature for 3 hours, poured into dilute HCl (2N, 100ml), extracted with ethyl acetate (3x100ml), dried ($MgSO_4$) and concentrated to a solid (2.1g) which was chromatographed on silica.

Elution with chloroform gave 6-cyano-2,2-dimethyl-4-(4-fluorophenylaminothiocarbonyl)-2H-1-benzopyran-3-ol (1.43g, 82%).
$^1H$ nmr ($CDCl_3$) 1.51 (s,6H), 7.03 (d, 1H, J=8.5Hz), 7.11-7.17 (m,2H), 7.37-7.40 (m,3H), 7.61 (s,1H), 8.69 (br s, 1H, disappears with $D_20$), 13.65 (br s, 1H, disappears with $D_20$).

| Accurate mass $C_{19}H_{15}FN_2O_2S$ requires | 354.0838 |
|---|---|
| found | 354.0838 |

Example 13

6-Cyano-2,2-dimethyl-4-(4-methoxyphenylaminocarbonyl)-2H-1-benzopyran-3-ol

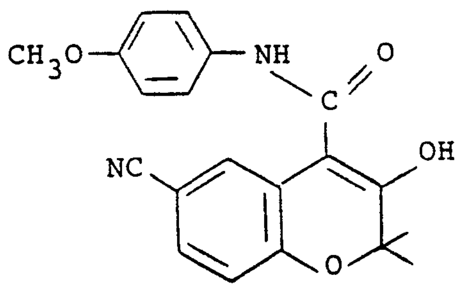

E13

6-Cyano-3,4-dihydro-2,2-dimethyl-2H-1-benzopyran-3-one (1g, 4.98mmol) in tetrahydrofuran (10ml) was added to a solution of potassium-t-butoxide (610mg, 5mmol) in tetrahydrofurn (15ml) at 0°C under $N_2$. The solution was stirred at ambient temperature for 45 minutes before the addition of 4-methoxyphenylisocyanate (0.645ml, 5mmol). The reaction mixture was stirred overnight at ambient temperature, poured into 2N HCl (50ml), extracted with chloroform (3 x 50ml), dried ($MgSO_4$) and concentrated to a solid (1.85g) which was chromatographed on silica. Elution with chloroform/ethyl acetate (90:10) gave 6-cyano-2,2-dimethyl-4-(4-methoxyphenylaminocarbonyl) -2H-1-benzopyran-3-ol (0.58g, 33%), m.p. = 158°C (EtOAc). $^1$H nmr ($CDCl_3$) δ: 1.49 (s,6H); 3.82 (s, 3H); 6.92 (d, 2H, J=9Hz); 7.02 (d, 1H, J=8.5Hz); 7.38 (d, 2H, J=9Hz); 7.40 (dd, 1H 3=2 and 8.5Hz); 7.46 (br, 1H, disappears with $D_2O$); 7.65 (d, 1H, J=2Hz); 14.16 (5, 1H, disappears with $D_2O$).

| | | | |
|---|---|---|---|
| Analysis: found: | C 68.80, | H 5.13, | N 7.93%. |
| $C_{20}H_{18}N_2O_4$ requires: | C 68.60, | H 5.18, | N 8.00% |

| | |
|---|---|
| Accurate mass theoretical | 350.1266 |
| observed | 350.1265 |

Example 14

6-Cyano-2,2-dimethyl-4-(4-methoxyphenylaminothiocarbonyl)-2H-1-benzopyran-3-ol

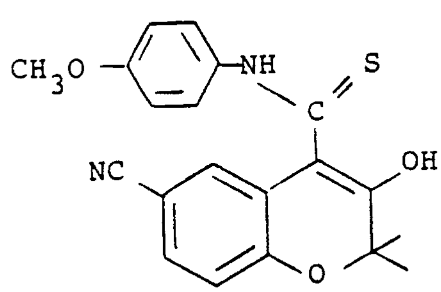

6-Cyano-3,4-dihydro-2,2-dimethyl-2H-1-benzopyran-3-one (1g, 4.98mmol) in tetrahydrofuran (10ml) was added to a solution of potassium t-butoxide (610mg, 5mmol) in tetrahydrofuran (15ml) at 0°C under $N_2$. The solution was stirred for 45 minutes at ambient temperature before the addition of 4-methoxyphenylisothiocyanate (0.7ml 5mmol). The reaction mixture was stirred at ambient temperature for 6 hours, poured into 2N HCl (50ml), extracted with $CHCl_3$ (3 x 50ml), dried ($MgSO_4$) and concentrated to a solid (1.8g) which was chromatographed on silica. Elution with chloroform gave 6-cyano-2,2-dimethyl-4-(4-methoxyphenylaminothiocarbonyl)-2H-1-benzopyran-3-ol (0.736g, 41%), melting observed at 169°C followed by resolidification and melting at 225°C (EtOAc).
$^1H$ nmr ($CDCl_3$) δ: 1.50 (s,6H), 3.82 (s, 3H); 6.97 (m, 3H); 7.36 (m, 3H); 7.61 (s, 1H); 8.74 (s, 1H, disappears with $D_2O$); 13.63 (s, 1H, disappears with $D_2O$).

| | | | |
|---|---|---|---|
| Analysis: found: | C 65.66, | H 4.99, | N 7.64%. |
| $C_{20}H_{18}N_2O_3S$ requires: | C 65.55, | H 4.95, | N 7.64% |

## Example 15

4-t-Butylaminothiocarbonyl-6-cyano-2,2-dimethyl-2H-1-benzopyran

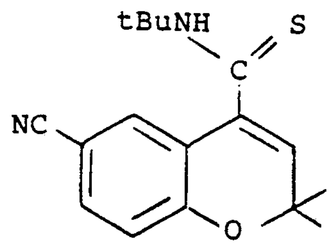

Trans-6-cyano-3,4-dihydro-2,2-dimethyl-4-t-butylaminothiocarbonyl-3-methylsulphonyloxy-2H-1-benzopyran (0.313g, 0.79mmol) was dissolved in THF (13ml) and cooled to 0°C after which potassium t-butoxide (96mg, 0.86mmol) was added. The solution was stirred for 1 hr then further potassium t-butoxide (90mg, 0.8mmol) added and the reaction stirred for a further 1 hr, poured into water and extracted with diethylether. The organics were dried ($MgSO_4$) and concentrated *in vacuo*. Chromatographic purification over silica ($CHCl_3$) yielded 4-t-butylaminothiocarbonyl-6-cyano-2,2-dimethyl-2-H-1-benzopyran (102mg, 47%) m.p. 187°C (EtOAc).
$^1H$ nmr ($CDCl_3$): δ 1.48 (s,6H), 1.64 (s,9H), 5.74 (s,1H), 6.85 (d, 1H, J=8.5Hz); 7.22 (brs, 1H); 7.41 (dd, 1H, J=8.5 and 2Hz); 7.71 (d, 1H, J=2Hz). Accurate Mass Found 300.1297; $C_{17}H_{20}N_2OS$ requires 300.1296

## Example 16

6-Cyano-2,2-dimethyl-4-phenylaminocarbonyl-2H-1-benzopyran

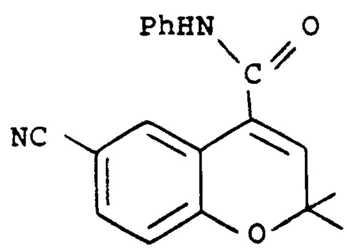

Triethylamine (1.44ml, 10.5mmol) was added to a solution of trans-6-cyano-3,4-dihydro-2,2-dimethyl-4-phenylaminothiocarbonyl-2H-1-benzopyran-3-ol (1.75, 5.18mmol) in chloroform (120ml) at 0°C. To the solution was added phosgene (1 equiv. as a solution in toluene) and the mixture stirred for 16 hrs. The reaction mixture was poured into water and the organics separated, dried ($MgSO_4$) and concentrated. The crude product was dissolved in THF (100ml) and potassium t-butoxide (610mg, 5.4mmol) added. The solution was stirred for 3 hrs, poured into water and extracted with ethyl acetate. The organics were separated, dried and concentrated. Chromotography over silica (hexane/ethylacetate 7:3) yielded 6-cyano-2,2-dimethyl-4-phenylaminocarbonyl-2H-1-benzopyran (170mg, 10%) m.p. 233.5-234°C.

$^1$H nmr (DMSO-$d_6$): δ 1.5 (s,6H); 6.41 (s,1H), 7.0 (d, 1H, J=8Hz), 7.10 (m,1H), 7.3 (m,2H) 7.65 (m,3H), 7.89 (d, 1H, J=2Hz), 10.32 (s, 1H).

Accurate Mass Found 304.1219; $C_{19}H_{16}N_2O_2$ requires 304.1212

Example 17

6-Cyano-2,2-dimethyl-4-methylaminocarbonyl-2H-1-benzopyran

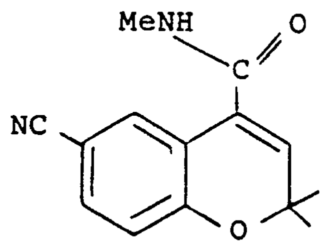

Potassium t-butoxide (1.0g, 8.94mmol) was added portionwise to a solution of trans-6-cyano-3,4-dihydro-2,2-dimethyl-4-methylaminothiocarbonyl-3-methanesulphonyloxy-2H-1-benzopyran (3.2g, 9.04mmol) in THF (100ml) at 0°C under $N_2$. The solution was stirred for 2 hrs and a further portion of potassium t-butoxide (1.0g, 8.94mmol) added and stirring continued for a further 1 hr. The mixture was poured into water, extracted with ether, dried ($MgSO_4$) and concentrated. Chromatography on silica (chloroform) yielded first 6-cyano-2,2-dimethyl-4-methylaminothiocarbonyl-2H-1-benzopyran (Example 4) (1.05g, 45%) followed by 700mg of a mixture with 6-cyano-2,2-dimethyl-4-methylaminocarbonyl-2H-1-benzopyran. Further purification of the mixture by chromatography over silica (ether) yielded 6-cyano-2,2-dimethyl-4-methylaminocarbonyl-2H-1-benzopyran (0.26g, 12%), m.p. 191-191.5°C.

$^1$H nmr ($CDCl_3$): δ 1.53 (s,6H); 3.00 (d, 3H, J=5Hz); 6.07 (s,1H); 6.10 (brs, 1H); 6.90 (d, 1H, J=8.5Hz); 7.48 (dd, 1H, J=8.5, 2Hz); 7.94 (d, 1H, 2Hz).

Accurate Mass Found 242.1058; $C_{14}H_{14}N_2O_2$ requires 242.1055

Example 18

2,2-Dimethyl-3-hydroxy-4-methylaminothiocarbonyl-2H-pyrano[3,2-c]pyridine

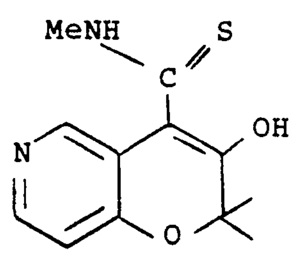

2,2-Dimethyl-3-oxo-2H-pyrano[3,2-C]pyridine (0.83g, 4.7mmol) was dissolved in dry THF (25ml) and cooled to 0°C whereupon methyl isothiocyanate (0.96ml, 14mmol) was added followed by the portionwise addition of potassium t-butoxide (0.556g, 4.9mmol) over 15min. The solution was stirred for 1.5 hrs poured into water and extracted with ethyl acetate. The organics were dried and concentrated to afford a gum which was chromatographed (ethyl acetate) to yield 2,2-dimethyl-3-hydroxy-4-methylaminothio-carbonyl-2H-pyrano[3,2-c] pyridine (0.23g, 19%) m.p. 190-191°C (ethylacetate).
$^1H$ nmr ($CDCl_3$): δ 1.5 (s,6H); 3.25 (s,3H); 6.84 (d, 1H, J=6Hz); 7.63 (brd, 1H, J=6Hz); 10.06 (brs, 1H); 12.1 (br, 1H).
Anal. Found C, 57.16; H, 5.69; N 10.67% $C_{12}H_{14}N_2O_2S$ requires C, 57.57; H, 5.64; N, 11.19%.
Accurate Mass Found 250.0775; $C_{12}H_{14}N_2O_2S$ requires 250.0776.

Example 19

3,4-Dihydro-2,2-dimethyl-6-ethyl-4-methylaminocarbonyl-2H-1-benzopyran

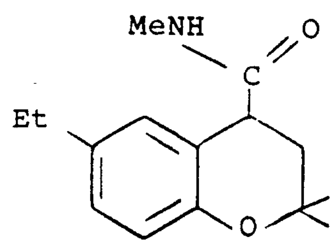

A solution of 3,4-dihydro-2,2-dimethyl-6-ethyl-2H-1-benzopyran-4-carboxylic acid (0.561g, 2.4mmol) in oxalyl chloride (1ml) was refluxed for 4hrs, cooled, concentrated and the residue dissolved in dichloromethane (10ml). To this solution was added methylamine solution (30ml, 40% solution in water). Removal of the organic solvent in vacuo and filtration gave 3,4-dihydro-2,2-dimethyl-6-ethyl-4-methylaminocarbonyl-2H-1-benzopyran (470mg, 79%) m.p. 159-160°C ($Et_2O$/hexane). $^1H$ nmr ($CDCl_3$): δ 1.20 (t, J=7.5Hz, 3H); 1.28 (s,3H); 1.34 (s,3H); 2.07 (dd, J=14, 7Hz,1H); 2.22 (dd, J=13.5,7Hz,1H); 2.57 (q, J=7.5Hz,2H); 2.82 (d, J=5Hz,3H); 3.69 (t, J=7.5Hz,1H); 5.72 (br,1H); 6.76 (d, J=8Hz,1H); 6.92 (brs,1H); 7.02 (dd, J=8, 2Hz,1H).

Accurate Mass Found 247.1569; $C_{15}H_{21}NO_2$ requires 247.1572.

Example 20

2,2-Dimethyl-6-pentafluoroethyl-4-phenylaminothio carbonyl-2H-1-benzopyran-3-ol

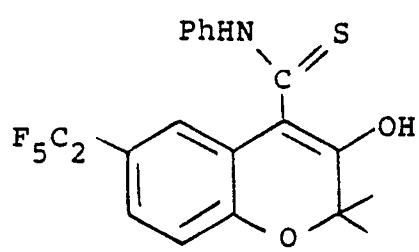

3,4-Dihydro-2,2-dimethyl-6-pentafluoroethyl-2H-1-benzopyran-3-one (200mg, 0.68mmol) was dissolved in THF (4ml) under nitrogen. The solution was cooled to 0°C, potassium t-butoxide (76mg, 0.68mmol) added

and the mixture stirred for 1.5 hrs. Phenyl isothiocyanate (92mg, 0.68mmol) was added and stirring continued for 3 hrs before pouring into 2N HHI (100ml). The mixture was extracted with ethylacetate, extracts washed with water, dried and evaporated to yield 2,2-dimethyl-6-pentafluoroethyl-4-phenylamino thiocarbonyl-2H-1- benzopyran-3-ol (183mg, 63%), as yellow crystals m.p. 127-128.5°C (hexane).
$^1$H nmr (CDCl$_3$): δ 1.52 (s,6H); 7.07 (d, J = 8.5Hz, 1H); 7.26-7.56 (m,7H); 8.78 (s,1H); 13.6 (s,1H).

| | | | |
|---|---|---|---|
| Anal. Found | C, 55.97; | H, 3.69; | N, 3.25%; |
| $C_{20}H_{16}NO_2SF_5$ requires | C, 55.94; | H, 3.76; | N, 3.26%. |

Example 21

2,2-Dimethyl-6-pentafluoroethyl-4-phenylaminocarbonyl-2H-1-benzopyran-3-ol

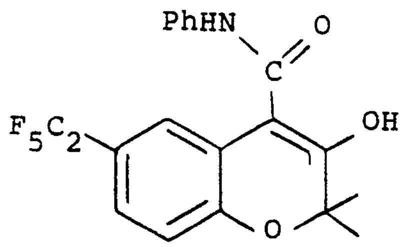

2,2-Dimethyl-6-pentafluoroethyl-4-phenylaminocarbonyl-2H-1-benzopyran-3-ol was prepared by the method of Example 20 but replacing phenylisothiocyanate with phenyl isocyanate. The compound m.p. 108-109°C (ether) was obtained in 45% yield after chromatography on silica (dichloromethane).
$^1$H nmr (CDCl$_3$): δ 1.53 (s,6H); 7.0-7.87 (m,9H); 14.03 (s,1H).

Example 22

6-Cyano-2,2-dimethyl-3-methoxy-4-phenylaminocarbonyl-2H-1-benzopyran

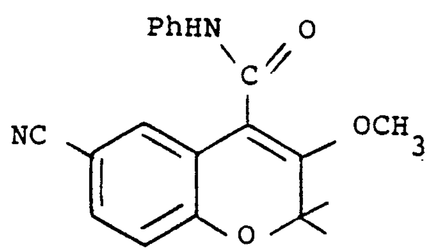

6-cyano-2,2-dimethyl-3-hydroxy-4-phenylaminocarbonyl-2H-1-benzopyran (150mg, 0.47mmol) was dissolved in dichloromethane (5ml). Trimethyloxonium tetrafluoroborate (75mg, 0.47mmol) and diisopropylethylamine (0.1ml, 0.47mmol) were added. The reaction mixture was stirred at room temperature for 4 hrs and then washed with sodium bicarbonate solution. The aqueous phase was extracted with chloroform and the combined organic phases dried and concentrated to give 170mg of a yellow oil which was chromatographed on silica (hexane 5/ethyl acetate 1) to give successively:
6-cyano-2,2-dimethyl-3-hydroxy-4-(methoxy-N-phenylimino)-2H-1-benzopyran (38mg, 24%).
$^1$H nmr (CDCl$_3$): δ 1.45 (s,6H); 3.71 (s,H); 6.98 (d, J = 8.25Hz,1H); 7.33 (m,6H); 7.91 (d, J = 1.93Hz,1H); 13.55 (br,1H);
6-cyano-3,4-dihydro-2,2,4-trimethyl-4-phenylamino carbonyl-2H-1-benzopyran-3-one (74mg, 48%).

$^1$H nmr ($CDCl_3$): δ 1.35 (s,3H); 1.63 (s,3H); 1.88 (s, 3H); 7.25 (m,7H); 7.69 (m,2H); 6-cyano-2,2-dimethyl-3-methoxy-4-phenylaminocarbonyl-2H-1-benzopyran (21mg, 13%). $^1$H nmr ($CDCl_3$): δ 1.51 (s,6H); 3.97 (s,3H); 6.88 (d, J=8.25Hz,1H); 7.22-7.8 (m,8H).

Example 23

3,4-Dihydro-2,2-dimethyl-6-ethyl-4-phenylaminocarbonyl-2H-1-benzopyran

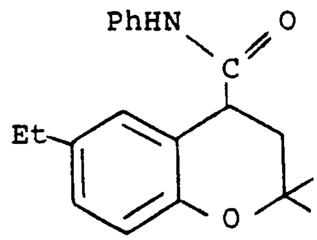

Oxalyl chloride (250μl, 2.76mmol) was added to a solution of 3,4-dihydro-2,2-dimethyl-6-ethyl-2H-1-benzopyran-4-carboxylic acid (0.43g, 1.84mmol) in dichloromethane (1.5ml) and the resultant orange solution was heated under reflux for 21 hr. Evaporation gave a brown oil which was redissolved in dichloromethane (3ml) and treated with aniline (177mg, 1.9mmol) and triethylamine (372mg, 3.68mmol). The solution was heated under reflux for 1 hr, cooled, diluted with ether, filtered and evaporated. The resulting oil was chromatographed on silica to give 3,4-dihydro-2,2-dimethyl-6-ethyl-4-phenylaminocarbonyl-2H-1-benzopyran (67mg, 12%) as an off-white solid m.p. 166-167°C ($CHCl_3$/hexane). $^1$H nmr ($CDCl_3$): δ 1.20 (3H,5, J=7.5Hz); 1.31 (3H,s); 1.38 (3H,s), 2.17 (2H,dd, J=13.5, 7Hz,); 2.31 (2H,dd, J=13.5, 8Hz); 2.58 (1H,t, J=7Hz); 6.82 (1H,d, J=8Hz); 7.03 (2H,brs); 7.07 (1H,dd, J=13.5, 8Hz); 7.31 (2H,t, J=8Hz); 7.43 (2H,t, J=8Hz); 7.45 (1H, s).

Pharmacological Data

1. Bronchodilator Activity

(a) Bronchodilation in vitro; guinea pig tracheal spiral preparations.

Male guinea pigs (300-600g) were stunned by a blow to the head and bled from the carotid artery. The trachea was exposed, dissected free of connective tissue, and transferred to oxygenated krebs solution at 37°C. Next, spirals (2 per trachea) were prepared by cutting the whole trachea spirally along its longitudinal axis and then dividing this spiral lengthwise. Each preparation was mounted, using silk thread, in a 10ml organ bath filled with krebs solution at 37°C and bubbled with 5% $CO_2$ with $O_2$. The resting tension of the preparations was set at 2g and changes in muscle tension were monitored isometrically by means of a UFI (2oz) force and displacement transducer (Ormed Ltd) connected to a Linseis pen recorder. All preparations were allowed to equilibrate for 60 minutes. During this equilibration period the preparations were washed by upward displacement at 15 minute intervals and, if necessary, the resting tension was readjusted to 2g using a mechanical micromanipulator system.

Once a steady resting tension had been obtained, the preparations were dosed cumulatively with the test compound ($10^{-8}$-$2x10^{-5}$M), and finally a maximum relaxation achieved by addition of $10^{-3}$M isoprenaline. The fall in tension evoked by the test compound was expressed as a percentage of the total relaxation evoked in the presence of $10^{-3}$M isoprenaline.

Appropriate concentration-relaxation curves were then constructed and values for potency ($IC_{50}$) were obtained.

The composition of Krebs solution is: sodium chloride 118.07mM, sodium hydrogen carbonate 26.19mM, potassium chloride 4.68mM, potassium orthophosphate 1.18mM, magnesium sulphate septahydrate 1.8mM and calcium chloride 2.52mM; pH ca. 7.45.

| Results | |
|---|---|
| Compound of Example No: | In vitro $IC_{50}$ value (M) |
| 2 | $6.4 \times 10^{-6}$ |
| 3 | $5.4 \times 10^{-6}$ |
| 4 | $1.4 \times 10^{-7}$ |
| 6 | $9.5 \times 10^{-6}$ |
| 8 | $2.6 \times 10^{-7}$ |
| 11 | $4.05 \times 10^{-6}$ |

2. Antihypertensive Activity

Blood Pressure Lowering Activity

Systolic blood pressures were recorded by a modification of the tail cuff method described by I.M. Claxton, M.G. Palfreyman, R.H. Pyser, R.L. Whiting, European Journal of Pharmacology, 37, 179 (1976). A W+W BP recorder, model 8005 was used to display pulses.Prior to all measurements rats were placed in a heated environment (33.5 ± 0.5°C) before transfer to a restraining cage. Each determination of blood pressure was the mean of at least 6 readings. Spontaneously hypertensive rats (ages 12-18 weeks) with systolic blood pressures >180 mmHg were considered hypertensive.

| Results | | | |
|---|---|---|---|
| Compound of: | dose in mg/kg | Time, post dose in hours | % Change in Systolic Blood Pressure (mm Hg) |
| Example 3 | 3 | 1 | - 33 ± 5 |
| | | 2 | - 32 ± 6 |
| | | 4 | - 24 ± 2 |
| | | (Initial BP 235 ± 0.6) | |
| Compound of: | | | |
| Example 4 | 3 | 1 | - 51 ± 5 |
| | | 2 | - 48 ± 3 |
| | | 4 | - 43 ± 3 |
| | | (Initial BP 232 ± 8) | |

## Claims

1. A process for the preparation of a compound of formula (I):

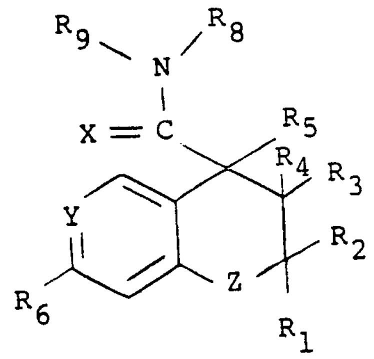

(I)

or, where appropriate, a pharmaceutically acceptable salt, ester or amide thereof, or a pharmaceutically acceptable solvate thereof
wherein:
X represents O or S;
Y represents N or $N^+$-$O^-$ or a moiety $CR_7$ wherein
$R_7$ is as defined below;
Z represents O, $CH_2$, NR, $S(O)_n$ or Z represents a bond;
R represents hydrogen, alkyl or alkylcarbonyl
$R_1$ and $R_2$ independently represent hydrogen or alkyl; or
$R_1$ and $R_2$ together represent a $C_{2-7}$ polymethylene chain;
$R_3$ represents hydrogen, hydroxy, alkoxy or acyloxy;
$R_4$ is hydrogen;
$R_5$ is hydrogen or $R_4$ and $R_5$ together represent a bond;
$R_6$ represents hydrogen, substituted or unsubstituted alkyl, alkoxy, $C_{3-8}$ cycloalkyl, hydroxy, nitro, cyano halo, formyl, carboxy, a group of formula $R^aT^1$-, $R^bR^cNT$- $R^aT^2NH$-, $R^dCO.O$-, $R^dCS.O$-, $R^d(OH)CH$-, $R^d(SH)$-CH-, $R^dC(=N.OH)$-, $R^dC(=N.NH_2)$- or alkenyl optionally substituted by alkylcarbonyl, nitro or cyano;
when Y represents $CR_7$, $R_7$ is independently selected from the variables mentioned above in relation to $R_6$ providing that at least one of $R_6$ and $R_7$ is other than hydrogen;
$R^a$ represents $R^d$ or $R^dO$- and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, hydroxy, halo, haloalkyl, nitro and cyano;
$R^b$ and $R^c$ each independently represent hydrogen, alkyl or alkylcarbonyl;
T represents a bond or $T^1$, $T^1$ represents -CS- or $T^2$ and
$T^2$ represents -CO-, -SO- or $-SO_2$- and
$R_8$ represents hydrogen, alkyl or substituted or unsubstituted aryl;
$R_9$ represents hydrogen or alkyl, substituted or unsubstituted aryl or $R_8$ and $R_9$ together represent a $C_{3-7}$ polymethylene group; and
n represents 0, 1 or 2.
which process comprises:
A) reacting a compound of formula (II):

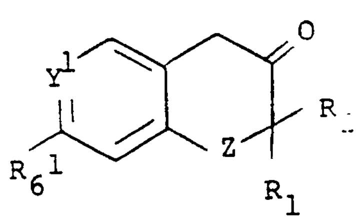

(II)

wherein $R_1$, $R_2$, and Z are as defined in relation to formula (I), Y' represents Y or a moiety convertible thereto and $R_6$' represents $R_6$ or a moiety convertible thereto, with a compound of formula (III):

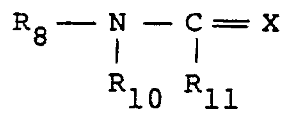

(III)

wherein $R_8$ and X are as defined in relation to formula (I),$R_{10}$ represents $R_9$ as defined above and $R_{11}$ represents a leaving group, or $R_{11}$ together with $R_{10}$ represents a bond; to provide a compound of formula (I) wherein $R_3$ represents hydroxy and $R_4$ together with $R_5$ represents a bond, and optionally thereafter converting such a compound into a compound of formula (I) wherein $R_3$ represents hydroxy and $R_4$ and $R_5$ both represent hydrogen and thereafter, if required, converting such a compound into a compound of formula (I) wherein $R_3$ represents hydrogen and $R_4$ and $R_5$ together represent a bond; or

B) for preparing a compound of formula (I) wherein $R_3$, $R_4$ and $R_5$ each represent hydrogen, or where appropriate a pharmaceutically acceptable salt, ester or amide thereof, or a pharmaceutically acceptable solvate thereof, by reacting a compound of formula (V):

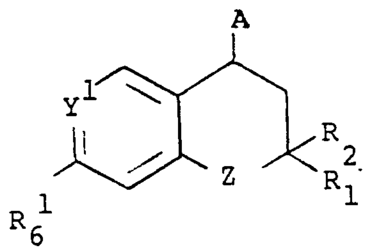

(V)

wherein $R_1$, $R_2$, $R_6'$, $Y^1$ and Z are as defined in relation to formula (II)and A represents a group or atom convertible to -CX.$NR_8R_9$,
with an appropriate reagent capable of converting A into -CX.$NR_8R_9$;
and thereafter, if required, carrying out one or more of the following optional steps:

(i) converting one compound of formula (I) into another compound of formula (I);

(ii) preparing a pharmaceutically acceptable salt, ester or amide of a compound of formula (I); or

(iii) preparing a pharmaceutically acceptable solvate of a compound of formula (I) or a solvate thereof.

2. A process according to claim 1, for preparing a compound of formula (I) wherein X represents S.

3. A process according to claim 1 or claim 2, for preparing a compound of formula (I) wherein Y is $CR_7$.

4. A process according to claim 3, for preparing a compound of formula (I) wherein one of $R_6$ and $R_7$ is hydrogen and the other is selected from substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d$-(OH)CH-, $R^d$(SH)CH-, $R^dC(=N.OH)$-, $R^dC(=N.NH_2)$- or alkenyl optionally substituted by alkylcarbonyl, nitro or cyano, wherein $R^a$, $R^b$, $R^c$, $R^d$, T, $T^1$ and $T^2$ are as defined in relation to formula (I) in claim 1.

5. A process according to any one of claims 1 to 4, for preparing a compound of formula (I) wherein $R_6$ represents hydrogen and $R_7$ represents alkyl fluoroalkyl, or cyano.

6. A process according to any one of claims 1 to 5, for preparing a compound of formula (I) wherein $R_7$ represents cyano.

7. A process according to any one of claims 1 to 6, for preparing a compound of formula (I) wherein $R_8$ or $R_9$ represents hydrogen, $C_{1-6}$ alkyl, phenyl or substituted phenyl,

8. A process according to any one of claims 1 to 7, for preparing a compound of formula (I) wherein $R_8$ represents hydrogen.

9. A process according to any one of claim 1 to 8, for preparing a compound of formula (I) wherein $R_9$ represents $C_{1-6}$ alkyl or phenyl.

10. A process according to any one of claims 1 to 9, for preparing a compound of formula (I) wherein Z represents O.

11. A process according to claim 1, for preparing a compound of formula (I) selected from the group consisting of:

6-cyano-2,2-dimethyl-4-methylaminothiocarbonyl-2H-1-benzopyran-3-ol;

6-cyano-3,4-dihydro-2,2-dimethyl-4-methylaminothiocarbonyl-2H-1-benzopyran-3-ol;

6-cyano-2,2-dimethyl-4-methylaminothiocarbonyl-2H-1-benzopyran;
4-t-butylaminothiocarbonyl-6-cyano-2,2-dimethyl-2H-1-benzopyran-3-ol;
4-t-butylaminothiocarbonyl-6-cyano-3,4-dihydro-2,2-dimethyl-2H-1-benzopyran-3-ol;
6-cyano-2,2-dimethyl-4-phenylaminothiocarbonyl-2H-1-benzopyran-3-ol;
6-cyano-2,2-dimethyl-4-phenylaminocarbonyl-2H-1-benzopyran-3-ol;
6-cyano-3,4-dihydro-2,2-dimethyl-4-phenylaminothiocarbonyl-2H-1-benzopyran-3-ol;
6-cyano-2,2-dimethyl-4-(4-fluorophenylaminothiocarbonyl)-2H-1-benzopyran-3-ol;
6-cyano-2,2-dimethyl-4-(4-methoxyphenylaminocarbonyl)-2H-1-benzopyran-3-ol;
6-cyano-2,2-dimethyl-4-(4-methoxyphenylaminothiocarbonyl)-2H-1-benzopyran-3-ol;
4-t-butylaminothiocarbonyl-6-cyano-2,2-dimethyl-2H-1-benzopyran;
6-cyano-2,2-dimethyl-4-phenylaminocarbonyl-2H-1-benzopyran;
6-cyano-2,2-dimethyl-4-methylaminocarbonyl-2H-1-benzopyran;
2,2-dimethyl-3-hydroxy-4-methylaminothiocarbonyl-2H-pyrano[3,2-c]pyridine;
3,4-dihydro-2,2-dimethyl-6-ethyl-4-methylaminocarbonyl-2H-1-benzopyran;
2,2-dimethyl-6-pentafluoroethyl-4-phenylaminothio carbonyl-2H-1-benzopyran-3-ol;
2,2-dimethyl-6-pentafluoroethyl-4-phenylaminocarbonyl-2H-1-benzopyran-3-ol;
6-cyano-2,2-dimethyl-3-methoxy-4-phenylaminocarbonyl-2H-1-benzopyran; and
3,4-dihydro-2,2-dimethyl-6-ethyl-4-phenylaminocarbonyl-2H-1-benzopyran; or a pharmaceutically acceptable salt, ester or amide thereof, or a pharmaceutically acceptable solvate thereof.

European Patent Office

# EUROPEAN SEARCH REPORT

Application Number

EP 90 30 5247

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-218373 (BEECHAM GROUP PLC) * pages 1 - 4, line 30 * | 1 | C07D311/58 C07D491/052 A61K31/44 //(C07D491/052 311:00,221:00) |
| A | EP-A-089781 (PFIZER INC.) * page 5, line 11 - page 6, line 16 * | 1 | |
| A | EP-A-214818 (BEECHAM GROUP PLC) * page 1, line 18 - page 7, line 6 * | 1 | |
| A | EP-A-205292 (BEECHAM GROUP PLC) * page 3, column 1, line 10 - page 5, column 6, line 25 * | 1 | |

TECHNICAL FIELDS SEARCHED (Int. Cl.5)

C07D311/00
C07D491/00

The present search report has been drawn up for all claims

| Place of search | Date of completion of the search | Examiner |
|---|---|---|
| BERLIN | 24 JULY 1990 | KYRIAKAKOU, G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document